# EUROPEAN PATENT SPECIFICATION

(11) **EP 4 016 077 B1**
(45) Date of publication and mention of the grant of the patent: **26.07.2023**
(21) Application number: 20306576.8
(22) Date of filing: 15.12.2020
(51) Int. Cl.: G01N 33/493

(54) **URINALYSIS DEVICE**
URINANALYSEVORRICHTUNG
DISPOSITIF D'ANALYSE D'URINE

(43) Date of publication of application: 22.06.2022
(73) Proprietor: Usense, 91300 Masy (FR)
(72) Inventor: OUERIEMMI, Amir, 91300 Massy (FR)
(74) Representative: Icosa

(56) References cited:
- WO-A2-2016/125165
- KR-A- 20190 012 392
- US-A1- 2014 150 535

## Description

### FIELD OF INVENTION

The present invention pertains to the field of urinalysis. In particular, the invention relates to a hand-held urinalysis device and methods for urinalysis.

### BACKGROUND OF INVENTION

Clinical urine tests, also known as urinalysis, are an examination of urine for certain biomarkers. Urinalysis is widely used for health check and/or diagnosis of diseases.

Currently, urinalysis is mostly performed in medical laboratories using different machines that measure different parameters or biomarkers concentration. This method has several disadvantages: logistic challenges, requires highly trained staff and expensive equipment. The main disadvantage of urinalysis performed in laboratories is the time to generate a result. Indeed, urine samples first need to be prepared and separated into several tubes to be tested in parallel on different machines measuring different parameters (optical or electrical) most often by chemical dosage. The main drawback of chemical dosage is the sample deterioration. Then, the results of each machine must be aggregated in a report and returned to the practitioner. The results are generated a long time (4h to 48h) after the sampling, causing a delayed diagnosis and generated unwanted and unnecessary stress to the patient. Known devices, as described in KR20190012392, US2014/150535 or WO2016/125165, do not allow for urinalysis by combining several measuring techniques.

Urinalysis is also performed by using urine test strips, in which the test results can be read as color changes. This allows a fast testing with a result generated in less than 10 minutes. However, this method requires lots of disposable consumables, more importantly proved to be inaccurate and to lead to false positive. Even if urine dipsticks tests can be considered as almost instantaneous diagnosis, these test strips may cause errors in diagnostics, creating the need for extra-testing and for worry and unnecessary follow-ups. Thus, there is a need for a device for point of care urinalysis allowing an instantaneous, thorough and reliable diagnosis by simultaneously measuring multiple parameters in a urine sample. Combining several measuring techniques such as visible spectrometry, near infrared spectrometry, autofluorescence spectrometry and conductimetry using one and only urinalysis reusable device overcomes the drawbacks mentioned hereabove.

### SUMMARY

This invention relates to a hand-held urinalysis device comprising a handle and a measuring head configured to be immersed in a urine sample, wherein the measuring head comprises:
a. a conductivity probe;
b. a lighting module configured to emit light in said urine sample; and
c. a multispectral optical sensor configured to receive light emitted by said urine sample and/or light transmitted through said urine sample.

In one embodiment, the hand-held urinalysis device further comprises a rechargeable battery. In one embodiment, the hand-held urinalysis device further comprises a connectivity system allowing for data transfer. In one embodiment, the conductivity probe is configured to measure conductivity with DC or AC current at two different frequencies, said frequencies being included in a range from about 1 Hz to 1 MHz. In one embodiment, the lighting module comprises a NIR-Vis light source and emits light over a range from 390 nm to 1100 nm. In one embodiment, the lighting module comprises a UV light source and emits UV light over a range from 270 nm to 400 nm. In one embodiment, the lighting module comprises a IR light source and emits IR light over a range from 800 nm to 2600 nm. In one embodiment, the multispectral optical sensor collects light over a range from 400 nm to 1100 nm. In one embodiment, the multispectral optical sensor collects light over a range from 800 nm to 2600 nm. In one embodiment, the hand-held urinalysis device further comprises a temperature sensor. In one embodiment, the hand-held urinalysis device further comprises a pH sensor.

This invention also relates to a method of urine analysis comprising the following steps:
i. collecting a urine sample in a container;
ii. immersing in said urine sample the hand-held urinalysis device according to the invention; and
iii. measuring the following physical properties of said sample:
   - conductivity; and
   - NIR-Vis spectrum, IR spectrum and/or fluorescence spectrum.

This invention also relates to a urinalysis system comprising a hand-held urinalysis device according to the invention; and a docking station comprising an electric charging module and/or a cleaning module. In one embodiment, the cleaning module comprises a disinfection solution.

This invention also relates to a method of urine analysis comprising the following steps:
i. collecting a urine sample in a container;
ii. immersing in said urine sample the measuring head of hand-held urinalysis device of urinalysis system according to the invention; and
iii. measuring the following physical properties of said sample:
   - conductivity; and
   - NIR-Vis spectrum, IR spectrum and/or fluorescence spectrum;
iv. placing the hand-held urinalysis device on the docking station of urinalysis system according to the invention, allowing for electric charging and/or cleaning; and
v. cleaning the measuring head with a disinfection solution.

### DEFINITIONS

In the present invention, the following terms have the following meanings:
- **"Autofluorescence spectrometry"** refers to the measurement of light emitted by a tissue or a solution after excitation of said tissue or solution with light at specific wavelength, in particular ultra-violet light.
- **"Conductimetry"** refers to the measurement of electrolytic conductivity of a solution.
- "**IR**" refers to infrared range of wavelengths, from 780 nm to 2600 nm.
- "**NIR**" refers to near infrared range of wavelengths, from 780 nm to 1100 nm.
- "**NIR-Vis**" refers to near infrared and visible range of wavelengths from 390 nm to 1100 nm.
- "**Near Infrared spectrometry**" refers to a quantitative measurement of light absorbance in near infrared range, i.e. of the ratio of the passed light with respect to the incident light in the near infrared range. This technique allows the detection of molecules absorbing low-energy radiation.
- "**UV**" refers to ultraviolet light, from 270 nm to 400 nm.
- "**Visible spectrometry**" refers to the characterization of the light absorption of a sample in visible range. When related to the extraction of quantitative information, one usually measures the intensity of light transmitted or reflected by the sample (I) and the intensity of a reference light (I₀) which can represent:
   - the intensity of light emitted by the source
   - the intensity of light incident on the sample
   - or the intensity of light transmitted or reflected by a reference sample Some calculations are thus performed and the ratio I/I₀ (usually called transmittance) or the decimal logarithm of this ratio (usually called absorbance) are calculated.

### DETAILED DESCRIPTION

The following detailed description will be better understood when read in conjunction with the drawings. For the purpose of illustrating, the device is shown in the preferred embodiments. It should be understood, however that the present invention is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

This invention relates to a hand-held urinalysis device, said device comprises a handle and a measuring head configured to be immersed in a urine sample, wherein the measuring head comprises:
a. a conductivity probe;
b. a lighting module configured to emit light in said urine sample; and
c. a multispectral optical sensor configured to receive light emitted by said urine sample and/or light transmitted through said urine sample.

The hand-held urinalysis device provides a non-invasive scan of urine samples based on four technologies: visible spectrometry, near infrared spectrometry, autofluorescence spectrometry and conductimetry. It allows a thorough physico-chemical characterization of a urine sample.

The multispectral optical sensor is configured to receive light transmitted through said urine sample such as visible and near infrared light, thus allowing the hand-held urinalysis device to perform visible spectrometry, near infrared spectrometry on a urine sample. Concerning visible spectrometry, it is possible to detect biomarkers such as, for example, minerals (e.g. Na, K, Ca, Mg, Cl, P), creatinine, urea, urine osmolality, urine specific gravity, uric acid, urine pH, ammonium, citrate, oxalate, albumin, total proteins, bilirubin, urobilinogen, red blood cells, white blood cells, ketones, glucose or presence of bacteria or crystals. Concerning near infrared spectrometry, it is possible to detect biomarkers such as, for example, minerals (e.g. Na, K, Ca, Mg, Cl, P), creatinine, urea, urine osmolality, urine specific gravity, uric acid, urine pH, ammonium, citrate, oxalate, albumin, total proteins, bilirubin, urobilinogen, red blood cells, white blood cells, ketones, glucose or presence of bacteria or crystals. Urine osmolality and urine specific gravity are biomarkers of hydration, very useful to determine how well the kidneys are working. Creatinine is also a biomarker that indicates about the proper functioning of kidneys.

The multispectral optical sensor is also configured to receive light emitted by said urine sample to anticipate autofluorescence of urine, thus allowing the hand-held urinalysis device to perform autofluorescence spectrometry on a urine sample. Fluorescence spectrometry allows the detection of biomarkers such as, for example, red blood cells, heavy metals, NADH, NADPH, FAD, elastin, collagens, tryptophan, porphyrins, riboflavin, or other endogenous fluorophores.

Finally, the conductivity probe allows the hand-held urinalysis device to perform conductimetry on a urine sample. The conductivity of urine arises mainly from the mobility of the constituents (hydrated ions) present in the sample and therefore gives a measure of the ability of the sample to conduct a charge applied to it. Thus, by measuring the conductivity of a urine sample, it is possible to determine the concentration of ions (for example Na⁺, K⁺, Ca²⁺, Mg²⁺, H⁺/CO₃⁻ or Cl⁻) in said sample.

Combining biomarkers detection by conductimetry and optical spectrometry is particularly advantageous as it allows for a thorough, and yet fast, scan of the urine sample, determining simultaneously the presence/absence and/or concentration of several biomarkers in a single urine sample. It also provides a better result, i.e. more precise, less false negative, than processing separately optical and electric measures. In addition, these measurements do not degrade the sample and can be repeated several times without impact on any future dosage. Finally, concomitant measurement of sample temperature allows to improve precision of optical and conductimetry measurement.

For example, near-infrared spectrometry and conductimetry are complementary to determine precisely mineral concentration (e.g. Na, K, Ca, Mg, Cl, P). Indeed, inorganic ions in aqueous solutions do not directly absorb NIR light but influence spectral patterns at specific wavelength through ion-water interactions. Similarly, urine saturation and crystallization (e.g. Calcium Oxalate) can be detected optically. Thus, optical spectrum brings both qualitative and quantitative information. This first estimation of each mineral concentration is completed by conductimetry measurement that reflects total concentration of cations and anions in solution, each ion having a specific molar conductivity. The measures of conductimetry at different frequencies allowing the precise determination of each ion concentration. Moreover, as the mobility of ions increase with temperature, a simultaneous measurement of temperature on top of optical spectrum and conductimetry allows an even more precise measure of mineral concentration.

Moreover, visible, NIR and IR spectra contain specific wavelengths that are heavily associated with similar urine biomarkers. Thus, combination of these spectral information significantly improves biomarker concentration prediction. For example, information can be found on osmolality below 700nm, between 800 and 850nm, around 1000nm, around 1150nm and above 1200nm, meaning all ranges contain information, sometimes redundant and sometimes not, allowing to improve osmolality measurement. Finally, fluorescence spectroscopy is used to identify specific biomarkers in combination with visible spectra. For example, hematuria can cause urine color change from light yellow to pink or red detectable in the visible spectrum. In that case, the presence of blood can be confirmed by fluorescence by measuring emission peak occurring at 450-520 nm.

To perform the biomarkers detection, the hand-held urinalysis device is immersed in a urine sample, activated to perform the measurements and then cleaned after use.

The urine sample can be provided by a human or an animal, e.g. cattle, sheep, pigs, horses or any other animal.

According to one embodiment, the hand-held urinalysis device has a cylindrical shape. In a particular configuration of his embodiment, said device has the shape of a pen. This allows the device to be hand-held, easy to use and easily carried from one place to another.

According to one embodiment, the measuring head comprises two walls hosting the sensor, lighting module and conductivity probe. In a particular configuration of this embodiment, the walls are opposed to one another (i.e. facing each other) and extending along the longitudinal axis of the hand-held urinalysis device. Preferably, the two walls of the measuring head are separated by an empty space, forming an optical path length. This allows urine to fill said empty space between the walls in order to submerge the sensor, lighting module and conductivity probe. The optical path length is particularly important because it determines the volume of sample that light will pass through. More information will be available for the measure if said volume is larger. Preferably, optical path length ranges between 1mm and 30mm

Preferably, the sensor, lighting module and conductivity probe are located on the internal surface of the walls, i.e. the surface facing towards the other wall.

In a particular configuration of this embodiment, the walls have a convex shape. Preferably, the walls are two halves of a cylinder extending from the handle and separated by an empty space. In this configuration, the sensor, lighting module and conductivity probe are located on the concave surface of the walls.

In a particular configuration of this embodiment, one wall has a higher thickness than the other, especially said thicker wall host the lighting module whereas the thinner wall host the multispectral optical sensor.

In a particular configuration of this embodiment, one wall has a higher thickness than the other, especially said thicker wall host the multispectral optical sensor whereas the thinner wall host the lighting module.

In a particular configuration of this embodiment, the conductivity probe is located nearer to the proximal end of the measuring head than the lighting module and the multispectral optical sensor, or the conductivity probe is located at the proximal end of the measuring head. Herein, the proximal end of the measuring head refers to the end connected with the handle, whereas the distal end of the measuring head refers to the end configured to be immersed in a sample.

In a particular configuration of this embodiment, the electrodes of the conductivity probe are located on the same wall. In an alternative configuration, electrodes of the conductivity probe are located on both walls, facing each other

In a particular configuration of this embodiment, the two electrodes of the conductivity probe are located on the same wall. In an alternative configuration, one electrode of the conductivity probe is located on each wall, i.e. the two electrodes face each other.

According to one embodiment, the hand-held urinalysis device further comprises an activation button (also called ON/OFF button) located at an extremity of the handle opposite from the measuring head.

According to one embodiment, the hand-held urinalysis device is configured to have at least 10% of its length immersed in a urine sample. In a particular configuration of this embodiment, the length of the hand-held urinalysis device immersed in the urine sample is ranging from 10 % to 50% of its total length.

According to one embodiment, the hand-held urinalysis device has a length ranging from 5 cm to 30 cm, preferably from 10 cm to 25 cm, more preferably from 10 cm to 20 cm.

According to one embodiment, the hand-held urinalysis device has a width ranging from 1 cm to 10 cm, preferably from 1 cm to 5 cm, more preferably from 2 cm to 4 cm.

According to one embodiment, the measuring head has a length ranging from 0,5 cm to 10 cm, preferably from 0,5 cm to 5 cm, more preferably from 0,5 cm to 3 cm. Thus, the hand-held urinalysis device is configured to be immersed in a urine sample in a length ranging from 0,5 cm to 10 cm, preferably from 0,5 cm to 5 cm, more preferably from 0,5 cm to 3 cm.

According to one embodiment, the measuring head has a length ranging from 3% to 80% of the length of the hand-held urinalysis device, preferably from 10% to 40% of the length of the hand-held urinalysis device, more preferably from 20% to 30% of the length of the hand-held urinalysis device.

According to one embodiment, the hand-held urinalysis device is not disposable. Said device is configured to be cleaned after use.

According to one embodiment, the conductivity probe is configured to measure direct current conductivity.

According to one embodiment, the conductivity probe is configured to measure conductivity at one or more different frequencies, said frequencies being included in a range from about 1 Hz to 1 MHz, preferably from about 1 Hz to 100kHz. In a preferred configuration of this embodiment, said frequencies are included in a range from about 10 Hz to 10 kHz. Having higher frequencies would lead to high power consumption and more complexity of the device.

According to one embodiment, the conductivity probe comprises two or more electrodes.

According to one embodiment, the lighting module comprises a NIR-Vis light source and emits light over a range from 390 nm to 1100 nm. In a particular configuration of this embodiment, the NIR-Vis light source is a LED (light emitting diode), a laser, a superluminescent LED (sLED), or a filament lamp.

According to one embodiment, the lighting module comprises a UV light source and emits UV light over a range from 270 nm to 400 nm, preferably from 365 nm to 400 nm. In a particular configuration of this embodiment, the UV light source is a UV LED (light emitting diode), a laser, a superluminescent LED (sLED), or a filament lamp.

According to one embodiment, the lighting module comprises a IR light source and emits IR light over a range from 800 nm to 2600 nm, preferably from 800 nm to 1350 nm.

According to one embodiment, the multispectral optical sensor collects light over a range from 400 nm to 1100 nm.

According to one embodiment, the multispectral optical sensor collects light over a range from 800 nm to 2600 nm.

According to one embodiment, the multispectral optical sensor comprises at least one light detector, preferably two or more light detectors. In a specific configuration of this embodiment, the multispectral optical sensor comprises a first light detector collecting light over a range from 400 nm to 1100 nm, i.e. a visible light collector, and a second light detector collecting light over a range from 800 nm to 2600 nm, i.e. an infrared light collector.

According to one embodiment, the hand-held urinalysis device further comprises a temperature sensor. The temperature sensor allows to measure the temperature of the urine sample which will then be converted into an electrical signal associated with said temperature. It also allows to normalize the optical and conductivity signals.

According to one embodiment, the hand-held urinalysis device further comprises a pH sensor. The pH sensor allows to measure pH of the urine sample which will then be converted into an electrical signal associated with said pH.

According to one embodiment, the sensors are integrated in the measuring head.

According to one embodiment, the hand-held urinalysis device comprises a handle and a measuring head configured to be immersed in a urine sample, wherein the measuring head comprises:
a. a conductivity probe;
b. a lighting module configured to emit light in said urine sample; and
   wherein the lighting module comprises a NIR-Vis light source emitting light over a range from 390 nm to 1100 nm, a UV light source emitting UV light over a range from 270 nm to 400 nm, and a IR light source emitting IR light over a range from 800 nm to 2600 nm;
c. a multispectral optical sensor configured to receive light emitted by said urine sample and/or light transmitted through said urine sample;
   wherein the multispectral optical sensor comprises a first light detector collecting light over a range from 400 nm to 1100 nm, and a second light detector collecting light over a range from 800 nm to 2600 nm;
d. a temperature sensor; and
e. a pH sensor.

According to one embodiment, the hand-held urinalysis device further comprises a rechargeable battery.

In a specific configuration of this embodiment, the rechargeable battery is configured to work during 24h without charging. In order to preserve the battery, a stand-by mode can be automatically activated when the device is not in use.

In another specific configuration of this embodiment, the rechargeable battery is configured to charge quickly, for example, the battery is fully charged after 1h.

In another specific configuration of this embodiment, the rechargeable battery has a size inferior to 2 cm x 6 cm.

According to one embodiment, the rechargeable battery is selected among lithium-ion battery, LiCFₓ battery, Li-FeS₂ battery, LiFePO₄ battery, Li-SO₂ battery, Li-I₂ battery, Li-Ag₂CrO₄ battery, Li-Ag₂V₄O₁₁ battery, Li-SVO battery, Li-CSVO battery, or lithium polymer battery. Preferably, the rechargeable battery is a lithium-based battery.

In an alternative embodiment, the hand-held urinalysis device further comprises a non-rechargeable battery, e.g. an alkaline battery.

In an alternative embodiment, the hand-held urinalysis device can be recharged by induction.

According to one embodiment, the hand-held urinalysis device further comprises a connectivity system allowing for data transfer. In a specific configuration of this embodiment, the hand-held urinalysis device is configured to communicate via wireless connection with a computing module such as for example a smartphone, a tablet or a computer, via wired or wireless (Bluetooth, wifi) connection.

According to one embodiment, the hand-held urinalysis device further comprises a display module configured to display data collected by the sensors/probe of the device and/or results obtained after data treatment.

This invention also relates to a method of urine analysis comprising the following steps:
i. collecting a urine sample in a container;
ii. immersing in said urine sample the hand-held urinalysis device according the invention; and
iii. measuring the following physical properties of said sample:
   - conductivity; and
   - NIR-Vis spectrum, IR spectrum and/or fluorescence spectrum.

The urine sample can be collected in any container.

Prior to immersion, the hand-held urinalysis device may be switched on. In a specific configuration, said device further comprises a first external LED, such as for example a blue LED, green LED or red LED, preferably a blue LED that indicates if said device is well switched on by emitting light.

Prior to immersion, the hand-held urinalysis device may also be connected to a computing module, such as a computer, a smartphone or a tablet, via a wireless connection.

To measure the physical properties of the urine sample, the immersed hand-held urinalysis device is activated by engaging the activation button. Upon immersion in a urine sample, the hand-held device performs the measurements when all sensor, conductivity probe, lighting module are submerged by urine. The start of the measurement can be ordered manually by the user or automatically when the device detects that all measuring elements are submerged by urine.

In a specific configuration, said device further comprises a second external LED, such as for example a blue LED, green LED or red LED, preferably a green LED that indicates if measurement was performed by emitting light and/or vibrating.

The method of the invention does not require that the sample is prepared, separated, or subjected to any treatment before immersing the measuring head of the hand-held device in said sample. Indeed, said measuring head can be immersed immediately after collection of the urine sample.

This invention also relates to a urinalysis system comprising a hand-held urinalysis device according to the invention; and a docking station comprising an electric charging module and/or a cleaning module.

The docking station is configured to wash, recharge and/or dry the hand-held urinalysis device.

After measurement in a urine sample, the hand-held urinalysis device is placed on the docking station where it can be recharged, and/or washed by immersing the measuring head in the cleaning module, and/or dried.

According to one embodiment, the docking station comprises an arm, preferably a mechanical articulated arm, to support the hand-held urinalysis device on said docking station and plunge the measuring head in the cleaning module.

According to one embodiment, the cleaning module comprises a disinfection solution (also called cleaning solution).

In a particular configuration of this embodiment, the disinfection solution is an aqueous solution comprising surfactants. This is particularly advantageous as said solution forms a foam, preferably a thermoformed foam, which helps to prevent the attachment of biological molecules on the hand-held urinalysis device.

In a particular configuration of this embodiment, the disinfection solution comprises didecyldimethylammonium chloride, polyhexamethylene biguanide hydrochloride, detergent complexes (polyalkoxylated fatty alcohol, lauryldimethylamine oxide), sequestering and dispersing agent.

In an alternative configuration of this embodiment, the disinfection solution refers to an alternance of water and solvent (e.g. ethanol), i.e. the measuring head is first plunged in water then in solvent, or first rinsed in water then solvent.

According to one embodiment, the docking station may further comprise a drying module. In a particular configuration of this embodiment, the drying module is an air-flowing module. This air-flowing module is particularly useful to evaporate the remains of disinfection solution after cleaning without making contact with the of hand-held urinalysis device.

Preferably, the docking station does not comprise a drying module and the hand-held urinalysis device is dried by ambient air without contact.

According to one embodiment, the electric charging module is a located in the arm. Said electric charging module may use contact (via a wire) or magnetic induction to recharge the battery of the hand-held urinalysis device.

According to one embodiment, the docking station further comprises a support for a tablet, or a tablet. In this embodiment, the tablet acts as a display and/or computing module for the results obtained after measurement in the urine sample.

According to one embodiment, the docking station may be configured to transport the hand-held urinalysis device. In this embodiment, said docking station is transportable, i.e. said docking station may be able to transform into a carrying case, the hand-held urinalysis device being inside said carrying case.

This invention also relates to a method of urine analysis comprising the following steps:
i. collecting a urine sample in a container;
ii. immersing in said urine sample the measuring head of hand-held urinalysis device of urinalysis system according to the invention; and
iii. measuring the following physical properties of said sample:
   - conductivity; and
   - NIR-Vis spectrum, IR spectrum and/or fluorescence spectrum;
iv. placing the hand-held urinalysis device on the docking station of urinalysis system according to the invention, allowing for electric charging and/or cleaning; and
v. cleaning the measuring head with a disinfection solution.

The disinfection solution is as described hereabove.

The washing step (v) is a fast, non-dangerous step that allows the user to reuse the device almost immediately.

This invention also relates to a use of the hand-held urinalysis device for urine analysis. Said urine can be human urine or animal urine.

This invention also relates to a use of the hand-held urinalysis device for analysis of a biological fluid, such as, for example, blood, sweat, tears, saliva, or breast milk.

While various embodiments have been described and illustrated, the detailed description is not to be construed as being limited hereto. Various modifications can be made to the embodiments by those skilled in the art without departing from the disclosure as defined by the claims.

### BRIEF DESCRIPTION OF THE DRAWINGS

Figure 1A is a schematic side-view of the hand-held urinalysis device of the invention.
Figure 1B is a schematic view of the hand-held urinalysis device of the invention.
Figure 2A is a schematic side-view of the hand-held urinalysis device of the invention comprising a conductivity probe, a lighting module and a multispectral optical sensor.
Figure 2B is a schematic side-view of the hand-held urinalysis device of the invention further comprising a temperature sensor.
Figure 3 is a schematic representation of the hand-held urinalysis device and docking station of the invention.

### ILLUSTRATIVE EMBODIMENTS OF THE INVENTION

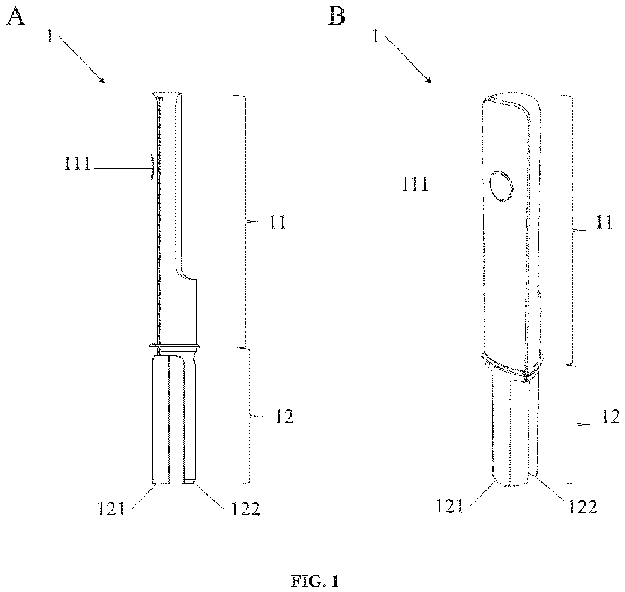

As illustrated on Figures 1A-B, the hand-held urinalysis device 1 comprises a handle 11 and a measuring head 12 configured to be immersed in a urine sample. Said measuring head 12 comprises a first wall 121 and a second wall 122, each wall being configured to host at least one of conductivity probe, lighting module 14 and multispectral optical sensor 13.

The hand-held urinalysis device 1 further comprises an activation button 111 located at an extremity of the handle 11. Upon immersion in a urine sample, the hand-held device 1 performs the measurements when all of conductivity probe, lighting module 14 and multispectral optical sensor 13 are submerged by urine. Advantageously, the start of the measurement can be ordered manually by the user by pressing the activation button 111.

The walls (121, 122) are facing each other and extending along the longitudinal axis of the hand-held urinalysis device 1. They are separated by an empty space, forming an optical path length.

This is particularly advantageous as it allows urine to fill said empty space between the walls (121, 122) in order to submerge the conductivity probe, lighting module 14 and multispectral optical sensor 13.

Furthermore, the optical path length created by the empty space separating the walls (121, 122) is particularly important because it determines the volume of sample that light will pass through. More information will be available for the measure if said volume is larger.

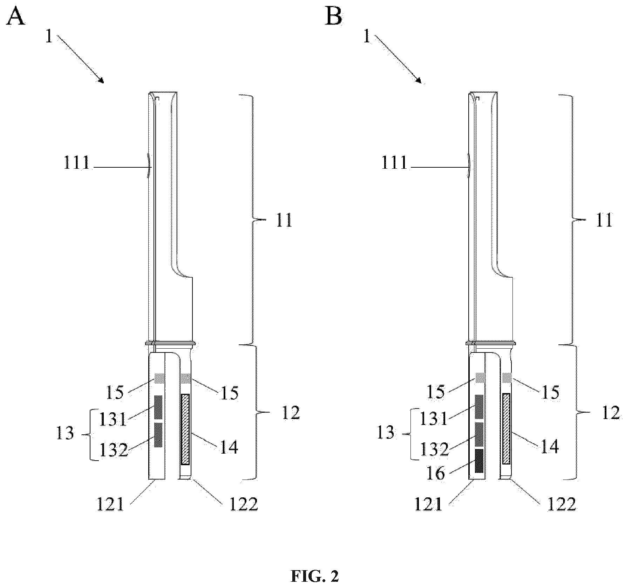

As illustrated on Figure 2A, the hand-held urinalysis device 1 comprises a handle 11 and a measuring head 12 configured to be immersed in a urine sample. Said handle 11 comprises an activation button 111. Said measuring head 12 comprises a first wall 121 and a second wall 122.

The first wall 121 is thicker than the second wall 122, and hosts:
- an electrode 15 of a conductivity probe; and
- a multispectral optical sensor 13 comprising an infrared light collector 131 and a visible light collector 132.

The second wall 122 hosts:
- a second electrode 15 of the conductivity probe; and
- a lighting module 14 configured to emit light in said urine sample.

Upon immersion in a urine sample, the hand-held urinalysis device 1 will be activated by, for example, pressing the activation button 111. Then, the lighting module 14 will emit light in said urine sample that will be collected by both infrared light collector 131 and visible light collector 132 depending on the wavelength of the light transmitted through the sample. Simultaneously to this optical measurement, the two electrodes 15 of the conductivity probe will measure the conductivity in said sample.

This is particularly advantageous as the hand-held urinalysis device 1 provides a non-invasive scan of urine samples based on four technologies: visible spectrometry, near infrared spectrometry, autofluorescence spectrometry and conductimetry. It allows a thorough physico-chemical characterization of a urine sample.

On Figure 2B, the hand-held urinalysis device 1 has the same configuration (same as Figure 2A) and further comprises a temperature sensor 16.

The temperature sensor 16 allows to measure the temperature of the urine sample which will then be converted into an electrical signal associated with said temperature. It also allows to normalize the optical and conductivity signals.

Advantageously, a simultaneous measurement of the temperature of the urine sample allows to improve precision of optical and conductimetry measurements.

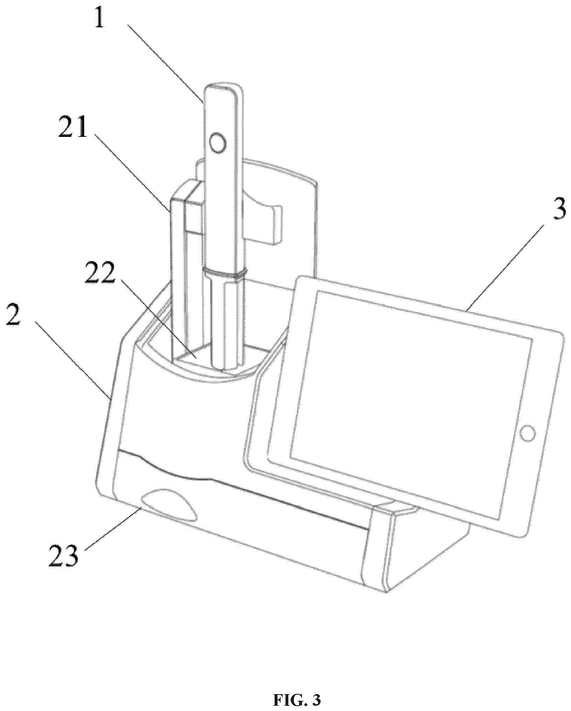

As illustrated on Figure 3, the docking station 2 can host a hand-held urinalysis device 1 supported by an articulated arm 21 and a table 3 for reading the results of the optical and conductimetry measurements. Said docking station 2 further comprises a cleaning module 22 configured to be recipient for a disinfection solution and a drawer 23 where the hand-held urinalysis device 1 can be stored after use. The articulated arm is configured to support the hand-held urinalysis device 1 for recharge purpose and to plunge said hand-held urinalysis device 1 in the disinfection solution contained in the cleaning module 22.

After measurement in a urine sample, the hand-held urinalysis device 1 is placed on the docking station 2 where it can be recharged, and/or washed by immersing the measuring head 12 in the cleaning module 22, and/or dried.

Advantageously, the docking station offers a unique station for recharging, washing and drying the hand-held urinalysis device 1, as well as a support for a tablet for an easy reading of the results.

### EXAMPLES

The present invention is further illustrated by the following examples.

### Example 1:

### Methods

Freshly collected urine samples were collected in 120 mL sterile urine containers without additives/preservatives. To evaluate the analytical performances of the hand-held urinalysis device of the invention, reference testing was performed by a central Lab with gold-standard methodologies.

The same samples were analyzed in parallel by using the hand-held urinalysis device of the invention to measure optical spectrum (visible spectrometry, near infrared spectrometry, autofluorescence spectrometry) and electrical conductivity. Numerical data were then processed by specific algorithms to determine concentrations values for each sample: on one side concentration values based on optical data, on the other side concentration values based on a combination of optical and electrical data.

The concentrations determined by the reference equipment were then used as control values and compared to both concentration values obtained by the hand-held urinalysis device: based on optical data and based on combination of optical and electrical data.

### Results

Table 1 shows correlations between biomarker concentration with gold-standard devices and results obtained by the hand-held urinalysis device with optical data, or with a combination of optical and electrical data.

**Table 1: Correlation between Gold Standard methodologies and the hand-held urinalysis device with or without electrical data.**

| | Gold Standard vs hand-held urinalysis device with optical data | Gold Standard vs hand-held urinalysis device with combined optical and electrical data |
|---|---|---|
| Osmolality | 0,92 | 0,99 |
| Oxalate | 0,87 | 0,95 |

We observed that compared to reference values we obtain a better precision with our hand-held device predictions when algorithms are used on a combination of optical and electrical data instead of only optical data.

### Example 2:

### Materials:

Freshly collected early morning urine samples were collected. A selection was made on urolithiasis patients in order to evaluate crystal presence in said urine samples. Each primary urine sample was collected in 120 mL sterile urine containers without additives/preservatives.

Urine samples are analyzed by the hand-held urinalysis device to characterize physico-chemical profile obtained by optical analysis (visible spectrometry, near infrared spectrometry, autofluorescence spectrometry) and electrical analysis (conductimetry) in order to determine sample concentrations.

### Results

Urines profiles measured with the hand-held device are highly specific and vary from one individual to another. Theses variations depend on parameters such as individual state of health and may be induced by pathologies such as urolithiasis which cause crystals to form in the urine.

When analyzing samples, the data obtained by the instrument varies according to the physico-chemical parameters. For example, the conductivity varies according to ionic concentration and the presence of crystals in the samples. In healthy people, conductivity varies from 11.49 to 16.85 mS.cm⁻¹.

The measured conductivity value performed by the hand-held device allowed to identify high crystal risk samples (conductivity > 25 mS.cm⁻¹) from healthy samples and to adapt algorithms predicting biomarker concentration. Crystal presence was confirmed by observing urine samples with a contrast microscope equipped with a polarized light device

### NUMERICAL REFERENCES

- 1 -: Hand-held urinalysis device
- 11 -: Handle
- 111 -: Activation button
- 12 -: Measuring head
- 121 -: First wall
- 122 -: Second wall
- 13 -: Multispectral optical sensor
- 131 -: Infrared light collector
- 132 -: Visible light collector
- 14 -: Lighting module
- 15 -: Electrode of conductivity probe
- 16 -: Temperature sensor
- 2 -: Docking station
- 21 -: Articulated arm
- 22 -: Cleaning module
- 23 -: Drawer
- 3 -: Tablet

## Claims

1. A hand-held urinalysis device (1) comprising a handle (11) and a measuring head (12) configured to be immersed in a urine sample, the measuring head (12) comprising:
a. a lighting module (14) configured to emit light in said urine sample;
**characterised in that** the measuring head (12) further comprises:
b. an electrical conductivity probe; and
c. a multispectral optical sensor (13) configured to receive light emitted by said urine sample and/or light transmitted through said urine sample.

2. The hand-held urinalysis device (1) according to claim **1,** further comprising a rechargeable battery.

3. The hand-held urinalysis device (1) according to claim **1** or **2,** further comprising a connectivity system allowing for data transfer.

4. The hand-held urinalysis device (1) according to any one of claims **1** to **3,** wherein conductivity probe is configured to measure conductivity at two different frequencies, said frequencies being included in a range from about 1 Hz to 1 MHz.

5. The hand-held urinalysis device (1) according to any one of claims **1** to **4,** wherein lighting module (14) comprises a NIR-Vis light source and emits light over a range from 390 nm to 1100 nm.

6. The hand-held urinalysis device (1) according to any one of claims **1** to **5,** wherein lighting module (14) comprises a UV light source and emits UV light over a range from 270 nm to 400 nm.

7. The hand-held urinalysis device (1) according to any one of claims **1** to **6,** wherein lighting module (14) comprises a IR light source and emits IR light over a range from 800 nm to 2600 nm.

8. The hand-held urinalysis device (1) according to any one of claims **1** to **7,** wherein the multispectral optical sensor (13) collects light over a range from 400 nm to 1100 nm.

9. The hand-held urinalysis device (1) according to any one of claims **1** to **8,** wherein the multispectral optical sensor (13) collects light over a range from 800 nm to 2600 nm.

10. The hand-held urinalysis device (1) according to any one of claims **1** to **9,** further comprising a temperature sensor (16).

11. The hand-held urinalysis device (1) according to any one of claims **1** to **10**, further comprising a pH sensor.

12. A method of urine analysis comprising the following steps:
i. collecting a urine sample in a container;
ii. immersing in said urine sample the hand-held urinalysis device (1) according to any one of claims **1** to **11**; and
iii. measuring the following physical properties of said sample:
• conductivity; and
• NIR-Vis spectrum, IR spectrum and/or fluorescence spectrum.

13. A urinalysis system comprising a hand-held urinalysis device (1) according to any one of claims **1** to **11**; and a docking station (2) comprising an electric charging module and/or a cleaning module (22).

14. The urinalysis system according to claim **13**, wherein the cleaning module (22) comprises a disinfection solution.

15. A method of urine analysis comprising the following steps:
i. collecting a urine sample in a container;
ii. immersing in said urine sample the measuring head (12) of hand-held urinalysis device (1) of urinalysis system according to any one of claims **13** to **14**; and
iii. measuring the following physical properties of said sample:
• conductivity; and
• NIR-Vis spectrum, IR spectrum and/or fluorescence spectrum;
iv. placing the hand-held urinalysis device (1) on the docking station (2) of urinalysis system according to any one of claim **13** to **14**, allowing for electric charging and/or cleaning; and
v. cleaning the measuring head (12) with a disinfection solution.

## Patentansprüche

1. Tragbare Urinanalysevorrichtung (1), umfassend einen Griff (11) und einen Messkopf (12), der so eingerichtet ist, dass er in eine Urinprobe eingetaucht werden kann, wobei der Messkopf (12) Folgendes umfasst:
a. ein Beleuchtungsmodul (14), das so eingerichtet ist, dass es Licht in der Urinprobe emittiert;
**dadurch gekennzeichnet, dass** der Messkopf (12) ferner Folgendes umfasst:
b. eine elektrische Leitfähigkeitssonde; und
c. einen multispektralen optischen Sensor (13), der so eingerichtet ist, dass er von der Urinprobe emittiertes Licht und/oder durch die Urinprobe durchgelassenes Licht empfängt.

2. Tragbare Urinanalysevorrichtung (1) nach Anspruch 1, ferner umfassend einen wiederaufladbaren Akku.

3. Tragbare Urinanalysevorrichtung (1) nach Anspruch 1 oder 2, ferner umfassend ein Verbindungssystem, das eine Datenübertragung ermöglicht.

4. Tragbare Urinanalysevorrichtung (1) nach einem der Ansprüche 1 bis 3, wobei die Leitfähigkeitssonde so eingerichtet ist, dass sie die Leitfähigkeit bei zwei verschiedenen Frequenzen misst, wobei die Frequenzen in einem Bereich von etwa 1 Hz bis 1 MHz liegen.

5. Tragbare Urinanalysevorrichtung (1) nach einem der Ansprüche 1 bis 4, wobei das Beleuchtungsmodul (14) eine NIR-Vis-Lichtquelle umfasst und Licht in einem Bereich von 390 nm bis 1100 nm emittiert.

6. Tragbare Urinanalysevorrichtung (1) nach einem der Ansprüche 1 bis 5, wobei das Beleuchtungsmodul (14) eine UV-Lichtquelle umfasst und UV-Licht in einem Bereich von 270 nm bis 400 nm emittiert.

7. Tragbare Urinanalysevorrichtung (1) nach einem der Ansprüche 1 bis 6, wobei das Beleuchtungsmodul (14) eine IR-Lichtquelle umfasst und IR-Licht in einem Bereich von 800 nm bis 2600 nm emittiert.

8. Tragbare Urinanalysevorrichtung (1) nach einem der Ansprüche 1 bis 7, wobei der multispektrale optische Sensor (13) Licht in einem Bereich von 400 nm bis 1100 nm sammelt.

9. Tragbare Urinanalysevorrichtung (1) nach einem der Ansprüche 1 bis 8, wobei der multispektrale optische Sensor (13) Licht in einem Bereich von 800 nm bis 2600 nm sammelt.

10. Tragbare Urinanalysevorrichtung (1) nach einem der Ansprüche 1 bis 9, ferner umfassend einen Temperatursensor (16).

11. Tragbare Urinanalysevorrichtung (1) nach einem der Ansprüche 1 bis 10, ferner umfassend einen pH-Sensor.

12. Verfahren zur Urinanalyse, umfassend die folgenden Schritte:
i. Sammeln einer Urinprobe in einem Behälter;
ii. Eintauchen der tragbaren Urinanalysevorrichtung (1) nach einem der Ansprüche 1 bis 11 in die Urinprobe; und
iii. Messen der folgenden physikalischen Eigenschaften der Probe:
• Leitfähigkeit; und
• NIR-Vis-Spektrum, IR-Spektrum und/oder Fluoreszenzspektrum.

13. Urinanalysesystem, umfassend eine tragbare Urinanalysevorrichtung (1) nach einem der Ansprüche 1 bis 11; und eine Dockingstation (2), die ein elektrisches Lademodul und/oder ein Reinigungsmodul (22) umfasst.

14. Urinanalysesystem nach Anspruch 13, wobei das Reinigungsmodul (22) eine Desinfektionslösung umfasst.

15. Verfahren zur Urinanalyse, umfassend die folgenden Schritte:
i. Sammeln einer Urinprobe in einem Behälter;
ii. Eintauchen des Messkopfes (12) der tragbaren Urinanalysevorrichtung (1) des Urinanalysesystems nach einem der Ansprüche 13 bis 14 in die Urinprobe; und
iii. Messen der folgenden physikalischen Eigenschaften der Probe:
• Leitfähigkeit; und
• NIR-Vis-Spektrum, IR-Spektrum und/oder Fluoreszenzspektrum;
iv. Platzieren der tragbaren Urinanalysevorrichtung (1) auf der Dockingstation (2) des Urinanalysesystems nach einem der Ansprüche 13 bis 14, um ein elektrisches Aufladen und/oder Reinigen zu ermöglichen; und
v. Reinigen des Messkopfes (12) mit einer Desinfektionslösung.

## Revendications

1. Dispositif d'analyse d'urine portatif (1) comprenant une poignée (11) et une tête de mesure (12) configurée pour être immergée dans un échantillon d'urine, la tête de mesure (12) comprenant :
a. un module d'éclairage (14) configuré pour émettre de la lumière dans ledit échantillon d'urine ;
**caractérisé en ce que** la tête de mesure (12) comprend en outre :
b. une sonde de conductivité électrique ; et
c. un capteur optique multispectral (13) configuré pour recevoir la lumière émise par ledit échantillon d'urine et/ou la lumière transmise à travers ledit échantillon d'urine.

2. Dispositif d'analyse d'urine portatif (1) selon la revendication 1, comprenant en outre une batterie rechargeable.

3. Dispositif d'analyse d'urine portatif (1) selon la revendication 1 ou 2, comprenant en outre un système de connectivité permettant le transfert de données.

4. Dispositif d'analyse d'urine portatif (1) selon l'une quelconque des revendications 1 à 3, dans lequel la sonde de conductivité est configurée pour mesurer la conductivité à deux fréquences différentes, lesdites fréquences étant comprises dans une plage d'environ 1 Hz à 1 MHz.

5. Dispositif d'analyse d'urine portatif (1) selon l'une quelconque des revendications 1 à 4, dans lequel le module d'éclairage (14) comprend une source de lumière NIR-Vis et émet de la lumière sur une gamme de 390 nm à 1100 nm.

6. Dispositif d'analyse d'urine portatif (1) selon l'une quelconque des revendications 1 à 5, dans lequel le module d'éclairage (14) comprend une source de lumière UV et émet de la lumière UV sur une gamme de 270 nm à 400 nm.

7. Dispositif d'analyse d'urine portatif (1) selon l'une quelconque des revendications 1 à 6, dans lequel le module d'éclairage (14) comprend une source de lumière IR et émet de la lumière IR sur une gamme de 800 nm à 2600 nm.

8. Dispositif portatif d'analyse d'urine (1) selon l'une quelconque des revendications 1 à 7, dans lequel le capteur optique multispectral (13) collecte la lumière sur une gamme de 400 nm à 1100 nm.

9. Dispositif portatif d'analyse d'urine (1) selon l'une quelconque des revendications 1 à 8, dans lequel le capteur optique multispectral (13) collecte la lumière sur une gamme de 800 nm à 2600 nm.

10. Dispositif d'analyse d'urine portatif (1) selon l'une quelconque des revendications 1 à 9, comprenant en outre un capteur de température (16).

11. Dispositif d'analyse d'urine portatif (1) selon l'une quelconque des revendications 1 à 10, comprenant en outre un capteur de pH.

12. Méthode d'analyse d'urine comprenant les étapes suivantes :
i. recueillir un échantillon d'urine dans un récipient ;
ii. immerger dans ledit échantillon d'urine le dispositif d'analyse d'urine portatif (1) selon l'une quelconque des revendications 1 à 11; et
iii. mesurer les propriétés physiques suivantes dudit échantillon :
• conductivité; et
• Spectre NIR-Vis, spectre IR et/ou spectre de fluorescence.

13. Système d'analyse d'urine comprenant un appareil d'analyse d'urine portatif (1) selon l'une quelconque des revendications 1 à 11; et une station d'accueil (2) comprenant un module de charge électrique et/ou un module de nettoyage (22).

14. Système d'analyse d'urine selon la revendication 13, dans lequel le module de nettoyage (22) comprend une solution de désinfection.

15. Méthode d'analyse d'urine comprenant les étapes suivantes :
i. recueillir un échantillon d'urine dans un récipient ;
ii. immerger dans ledit échantillon d'urine la tête de mesure (12) du dispositif d'analyse d'urine portatif (1) du système d'analyse d'urine selon l'une quelconque des revendications 13 à 14; et
iii. mesurer les propriétés physiques suivantes dudit échantillon :
• conductivité; et
• Spectre NIR-Vis, spectre IR et/ou spectre de fluorescence;
iv. placer le dispositif d'analyse d'urine portatif (1) sur la station d'accueil (2) du système d'analyse d'urine selon l'une quelconque des revendications 13 à 14, permettant une charge électrique et/ou un nettoyage; et
v. nettoyer la tête de mesure (12) avec une solution de désinfection.
